# FASCICULE DE BREVET EUROPEEN

(11) **EP 0 504 184 B1**
(45) Date de publication et mention de la délivrance du brevet: **04.09.1996**
(21) Numéro de dépôt: 90917709.9
(22) Date de dépôt: 05.12.1990
(51) Int. Cl.: A01N 33/14, A01N 59/00, A61L 2/16, A61K 7/40, A61K 31/18, A61K 31/155

(54) **AGENT ACTIF POUR COMBATTRE LES VIRUS DU GROUPE RETROVIRUS; COMPOSITIONS LE CONTENANT ET LEURS UTILISATIONS**
WIRKSTOFF ZUR BEKÄMPFUNG VON VIREN DER RETROVIRENGRUPPE, DIESEN ENTHALTENDE ZUSAMMENSETZUNGEN UND DEREN VERWENDUNGEN
ACTIVE AGENT AGAINST RETROVIRUS GROUP VIRUSES, COMPOSITIONS CONTAINING SAME AND THEIR USE

(30) Priorité: 06.12.1989 EP 89203106
(43) Date de publication de la demande: 23.09.1992
(73) Titulaire: PREVISAN S.A., Luxembourg (LU)
(72) Inventeur: VANDEVELDE, Michel, B-1301 Bierges (BE); Margery, Hélène, B-1301 Bierges (BE)
(74) Mandataire: Claeys, Pierre
(86) Numéro de dépôt international: EP9002111
(87) Numéro de publication internationale: WO9107876

(56) Documents cités:
- FR-A- 2 112 257
- GB-A- 421 006
- US-A- 2 073 256
- US-A- 2 157 831
- US-A- 2 275 593
- US-A- 3 577 532
- "WHO AIDS series", edition 2, 20 novembre 1989, World Health Organization, Geneva, CH; "Guidelines on sterilization and disinfection method effective against human immunodeficiency virus (HIV)", voir pages 7-8, chapitre "Decontamination of environmental surfaces with chlorine-releasing compounds"

## Description

La présente invention est relative à l'utilisation d'une composition de désinfection contenant pour combattre les virus du groupe rétrovirus, en particulier les virus d'immunodéficience humaine VIH, au moins un agent actif sous la forme d'un composé du groupe des chloroamines organiques. Elle concerne en outre l'utilisation de cet agent et de ces compositions.

Les rétrovirus sont définis suivant l'invention comme des virus dont le matériel génétique porté par une chaîne d'acide ribonucléique exige pour sa reproduction le passage sous forme d'acide désoxyribonucléique par l'intermédiaire d'un groupe d'enzymes appelées les transcriptases inverses.

Ces virus infectent le monde végétal et animal ainsi que l'être humain. Une liste non exhaustive de ces virus est donnée dans J.M. HURAUX et cons., Virologie, Flammarion Médecine-Sciences, 1985, Paris. Nombre de ces virus ont montré leur pouvoir oncogène chez l'animal et des études récentes montrent le même risque chez l'être humain.

Ces virus responsables de cancer chez l'animal servent de modèles pour la recherche de molécules susceptibles d'enrayer les maladies humaines.

C'est environ 70 ans après la découverte du virus du Sarcome de Rous (maladie de la souris) que sera découvert le premier rétrovirus responsable de la leucémie humaine.

Ces virus (HTLVI et HTLVII) semblent apparentés à des espèces virales infectant le singe et la maladie n'est observée que dans certaines régions du monde. L'évolution de la maladie est rapidement mortelle et aucun traitement n'est connu.

Par ailleurs, certains rétrovirus n'engendrent pas de cancer, mais d'autres maladies (encéphalite, anémie, pneumonie, par exemple chez l'animal) et notamment un Syndrome Immunodéficitaire acquis (SIDA) chez l'animal et chez l'homme.

La dénomination internationale de VIH a été acceptée pour les virus humains responsables de ce syndrome. Ceux-ci sont capables de mutations et l'on compte déjà 4 virus différents identifiables. De même les virus responsables d'immunodéficience chez le singe ont pris la dénomination de VIS.

Bien entendu, le problème majeur provient de l'émergence du SIDA humain et de sa rapide propagation a travers le monde.

Cette maladie en compromettant les défenses du malade permet l'émergence de maladies opportunistes ainsi que d'un cancer particulier (KAPOSI) entraînant la mort des patients.

A ce jour, un seul traitement est connu qui prolonge la survie des patients, sans permettre toutefois la guérison. Ce traitement comprend l'administration de 3'-azido-3'-désoxythimidine (v. EP-A-196185). Cette substance s'avère très toxique et très coûteuse. Par ailleurs, il semble déjà établi que le virus devient résistant à cette molécule à plus ou moins long terme, notamment après environ 12 à 18 mois (SCHULHAFER E. et cons., Acquired immuno-deficiency syndrome..., IN VIVO 3 (2) : 61-78 (1989)).

On a également considéré l'utilisation, comme médicament contre les rétrovirus, de certains dérivés de benzidine éventuellement substitués par de l'halogène (FR-A-2612515). Cependant, dans ce document il y a uniquement une simple affirmation concernant l'activité de ces composés.

Les modes de transmission des virus ont été établis lorsqu'il y avait eu contact sanguin direct et contact au travers de plaies par du matériel infecté. Le risque de transmission par des objets et notamment du matériel médical infecté est non négligeable.

Par ailleurs, le mode de transmission sexuel et la transmission aux enfants par le lait maternel infecté sont aussi établis ce qui montre le passage possible de particules infectantes à travers des muqueuses saines (P. LEPAGE et cons., Postnatal Transmission of HIV from Mother to Child, The Lancet, August 15, 1987, p. 400; C.J. MILLER et cons., Genital Mucosal Transmission of Simian Immunodificiency Virus, Journal of Virology, Oct. 1989, p. 4277-4284).

Il semble de plus en plus probable qu'il puisse exister une inoculation du virus par simple contact, c'est-à-dire à travers la peau ou une muqueuse. Certains chercheurs ont émis l'opinion que les virus inoculés sembleraient alors connaître une phase de réplication pendant laquelle ils restent dans la sphère de la muqueuse ou de la peau du porteur. Cette phase pourrait se prolonger pendant plusieurs mois. Ce ne serait que dans une seconde phase que les virus et/ou ses constituants dissémineraient à partir de la muqueuse (R. ZITTOUN, Syndrome Immuno Déficitaire Acquis, Doins Editeurs, Paris, 1986, p. 183-184).

Ainsi la mise au point de molécules permettant la désinfection de surfaces et d'objets inanimés ainsi que de matières et produits venant au contact des muqueuses et de la peau s'avère nécessaire pour l'éradication de la maladie. Il est indispensable d'empêcher dans la mesure du possible la transmission des rétrovirus entre un porteur et une personne saine. Enfin, avantageusement, il serait souhaitable de pouvoir mettre au point des compositions thérapeutiques actives, si possible au niveau systémique, et en tout cas au niveau local, notamment pendant la phase dite "muqueuse" de l'infection.

On connaît déjà des compositions et un procédé de désinfection à base d'oligosaccharides ou de polysaccharides naturels ou synthétiques ayant au moins un groupe S-oxoacide (v. EP-A-285357). Il résulte toutefois clairement des exemples donnés que, même si ces compositions sont actives contre les rétrovirus, elles laissent toujours subsister une partie des virus présents, ce qui, à terme, présente l'énorme danger de voir l'apparition d'une population de virus résistants, encore plus dangereux.

Dans une demande de brevet européenne n° 88870139.8, on a déjà prévu des produits susceptibles d'entrer localement en contact avec la peau, les muqueuses ou des sécrétions corporelles, ces produits comprenant un agent actif pour combattre les virus du groupe rétrovirus, par exemple de la suramine sodique.

On a par ailleurs examiné l'action vis-a-vis des rétrovirus des désinfectants chimiques courants, tels que l'éthanol, le glutaraldéhyde, l'hypochlorite de sodium, la formaline, la β-propiolactone, l'alcool dénaturé (V.B. SPIRE et cons., Inactivation of Lymphadenopathy associated virus by chemical disinfectants, The Lancet, October 20, 1984, p. 899-901; P.J.V. HANSON et cons., Chemical inactivation of HIV on surfaces, Br. Med. J., 1989, 298 : 862-4 ; L. RESNICK et cons., Stability and inactivation of HTLV-III/LAV under clinical and laboratory environments, JAMA, 1986, vol. 255, 14; L.S. MARTIN et cons., Disinfection and inactivation of the human T lymphotropic Virus type III/lymphadenopathy-associated virus, The Journal of Infectious Diseases, Vol. 152, n° 2, 1985).

Il ressort de ces examens que la plupart des désinfectants utilises en milieu hospitalier sont inefficaces ou peu efficaces vis-à-vis des rétrovirus VIH, et donc potentiellement dangereux. Ceux qui se sont montrés les plus efficaces demandent des temps de contact de 10 minutes au minimum, ce qui est matériellement difficile en cas de nettoyage de sols, de tables, par exemple. Enfin, certains de ces désinfectants semblent perdre leur efficacité en présence de milieux protéiques ou sont inapplicables s'ils doivent ou risquent de venir au contact de la peau ou des muqueuses d'un corps vivant, étant donné leur agressivité chimique à la forte concentration à laquelle on estime devoir les utiliser (V. WHO AIDS series, éd. 2, 1989, pages 7-8).

Il ressort enfin de la littérature que la plupart des essais effectués sur des composés pour examiner leur activité vis-à-vis des virus VIH se limitent à l'examen de l'activité enzymatique du virus, c'est-à-dire de la transcriptase inverse. Ce genre de test n'est pas très sensible; en effet, cette activité enzymatique n'est détectable que si un grand nombre de particules infectantes est présent. Ce test ne permet pas de détecter la destruction ou la disparition du virus inactivé.

La présente invention a par conséquent pour but de mettre au point une composition de désinfection telle que citée au début qui soit capable d'agir radicalement sur les rétrovirus, avec une élimination complète de ceux-ci, tout en maintenant son activité pendant une longue période de temps. Avantageusement, l'agent conservera son potentiel d'action en milieu cellulaire, en milieu aqueux, ainsi qu'en présence de milieux organiques, en particulier protéiques. Sa toxicité sera de préférence peu élevée. Sous une forme préférentielle, l'action germicide sera très rapide envers les virus VIH.

Il est évident que, selon ses applications, que ce soit dans la désinfection de sols, ou dans la désinfection de déchets organiques, par exemple provenant de laboratoires d'analyse, l'agent actif devra répondre à des exigences différentes de solubilité, de toxicité ou de stabilité.

On a à présent découvert que, d'une manière surprenante, certains composés chlorés sont capables de jouer de manière particulièrement efficace et radicale le rôle de l'agent actif recherché. Pour résoudre les problèmes posés, il est donc prévu suivant l'invention, l'utilisation d'une composition présentant les particularités caractéristiques de l'une ou l'autre des revendications 1 et 2.

Les dérivés N-chloro d'amines condensées provenant de dérivés de guanidine, utilisés à titre d'agents actifs dans une telle composition, sont des composés organiques chlorés bien connus, notamment pour leur libération stable et durable de chlore disponible (S.S. BLOCK, Disinfection, Sterilization, and Preservation, 3è éd., LEA & FEBIGER, 1983, Philadelphie, p. 173).

La chloroazodine, utilisée suivant l'invention à titre d'agent actif dans une composition de désinfection, est également connue depuis longtemps comme désinfectant (voir US-A-2073256 et GB-A-421006).

L'utilisation de ces substances pour combattre efficacement et rapidement les virus, en particulier les rétrovirus, n'est cependant ni décrite, ni suggérée dans ces publications antérieures.

Certains de ces composés organiques sont bien solubles dans l'eau ou leur solubilité peut être améliorée par l'addition d'adjuvants courants a cet effet. La chloroazodine a un très large spectre d'action sur les organismes pathogènes à des concentrations non toxiques pour les cellules. Elle ne semble pas ou peu affectée par la présence de protéines.

La chloroazodine en particulier a une action stable et durable vis-à-vis des rétrovirus VIH, mais en outre cette action est radicale à 100 % et elle a lieu en un temps très bref, de l'ordre de la minute, et cela à des concentrations extrêmement faibles.

Comme support approprié, on peut avantageusement prévoir de l'eau ou un quelconque autre solvant approprié comme véhicule dans lequel l'agent actif est en solution. D'autres agents désinfectants ou adjuvants courants peuvent être prévus éventuellement en supplément.

En particulier, une composition contenant de la chloroazodine et du cetrimide ou respectivement un laurylsulfate s'est avérée posséder un spectre d'une largeur inattendue contre non seulement les bactéries et les virus, mais aussi contre des champignons.

Suivant l'invention, on prévoit l'utilisation des compositions de désinfection précitées pour la désinfection, vis-à-vis des virus du groupe rétrovirus, en particulier des virus d'immunodéficience humaine VIH, d'objets inanimés.

Par désinfection vis-à-vis de rétrovirus, il faut entendre non seulement la destruction des organismes pathogènes, mais aussi l'inhibition de leur pénétration dans les cellules vivantes.

Comme objets inanimés à désinfecter, on peut prévoir, dans l'utilisation suivant l'invention, par exemple :
- des matériels sanitaires en matière plastique, textile ou caoutchouteuse : ouate, coton absorbant, gaze, bandages, papier hygiénique, films d'emballage, etc.
- des instruments et appareils médicaux, vétérinaires et de dentisterie : seringues, canules, sondes, pinces, ciseaux, trousses de lavage d'estomac, tables de chirurgie, bassins, etc...
- des vêtements médicaux, vétérinaires ou de dentisterie : gants, chemises, serviettes, etc...
- des objets nécessitant une manipulation dans le domaine de l'alimentation : biberons, casseroles, bouteilles ou boîtes, notamment pour des boissons, etc...
- des instruments de cosmétologie : matériel et outillage de coiffure, de manucure, de pédicure, d'esthéticien, etc...
- des véhicules sanitaires : ambulances, tables roulantes, etc...
- des surfaces de sol et de paroi : locaux, en particulier en milieu hospitalier, etc...
- des appareils sanitaires et hygiéniques : lavabos, pannes, cuvettes, baignoires, etc...
- des boissons : eaux nécessaires à des boissons, lait, etc....
- des eaux de piscine.

On peut aussi prévoir la désinfection des excréments, des résidus d'analyses de laboratoire et notamment d'échantillons prélevés du corps humain ou animal, par exemple en vue d'une analyse.

Il est avantageux de prévoir, suivant l'invention, que les compositions cosmétiques contiennent également au moins un agent actif suivant l'invention. Dans ce cas évidemment, divers supports et adjuvants courants dans ce domaine peuvent être mis en application.

On peut prévoir une utilisation particulière d'un agent actif ou d'une composition active suivant l'invention dans ou sur des produits susceptibles d'entrer en contact avec la peau ou les muqueuses d'un corps humain ou animal, éventuellement porteur de virus. Dans ou sur certains de ces produits, comme des gants de chirurgien ou de dentiste, des pessaires, des condoms, etc..., en plus de son action de désinfection, l'agent ou la composition active peut avantageusement former un milieu de barrière à la transmission des rétrovirus depuis le porteur vers une personne saine. On peut par exemple prévoir de lubrifier un condom en matière caoutchouteuse à l'aide d'une vaseline renfermant un agent actif suivant l'invention. On peut aussi, pour des gants de chirurgien, prévoir deux pellicules caoutchouteuses entre lesquelles une poudre d'amylase amylopectine par exemple contenant un agent actif suivant l'invention est enfermée. Dans ce dernier cas, la poudre désinfectante, servant de barrière, n'est donc pas elle- même en contact direct avec la peau.

L'invention a également pour objet d'utiliser au moins un derivé N-chloro d'amines condensées provenant le dérivés de guanidine ou de la chloroazodine pour la fabrication d'une composition thérapeutique à mettre en oeuvre dans le traitement ou la prophylaxie des infections par les virus du groupe rétrovirus, en particulier pour les infections par les virus d'immunodéficience humaine VIH. De préférence, on peut utiliser de la chloroazodine dont l'activité contre les virus VIH est totale déjà à une concentration extrêmement faible de l'ordre de 1 mg/ml à 300 µg/ml, de préférence de 660 µg/ml à 330 µg/ml.

Par désinfection, on peut aussi entendre ici une désactivation intracellulaire qu'elle soit enzymatique, ou autre.

Les composés organiques suivant l'invention sont susceptibles de conserver leur stabilité en milieu cellulaire, d'être compatibles pour l'organisme vivant et non toxiques pour les cellules. La présence de protéines n'affecte pas sensiblement leur action de libération lente et stable de chlore disponible. On peut notamment prévoir la fabrication, à partir par exemple de chloroazodine, de compositions locales désinfectantes, par exemple pour la désinfection de plaies, d'abcès, ou de parties de la peau et des muqueuses. On peut même envisager, étant donné l'excellente stabilité de la chloroazodine en milieu protéique, son utilisation pour la fabrication de médicaments pour le traitement systémique du Sida.

Les composés organiques suivant l'invention présentent, comme on l'a déjà dit, l'avantage d'offrir un large spectre d'action germicide et ils représentent par conséquent déjà une défense appréciable contre la présence d'agents pathogènes ou allogènes, autres que les rétrovirus VIH.

Cette dernière propriété est très importante non seulement par la large action de désinfection obtenue, mais aussi parce qu'elle peut être utile au porteur du virus lui-même. En effet, celui-ci doit éviter autant que possible toute activation de ses cellules lymphocytaires. Cette activation, chez le porteur de VIH, a pour effet la réplication du virus et la prolifération de ce dernier dans ses cellules. Un porteur de VIH a donc tout avantage a suivre des habitudes de vie hygiéniques qui écartent au maximum tout danger d'activation des cellules infectées, et par conséquent une réaction immunitaire de son organisme.

L'utilisation des compositions suivant l'invention lui offre la possibilité de se désinfecter, mais aussi en supplément celle de le protéger contre des réactions immunitaires d'une autre origine.

L'invention va à présent être décrite de manière plus détaillée à l'aide de quelques exemples de réalisation non limitatifs.

### Exemple 1

### Poudre désinfectante pour le lavage

| | |
|---|---|
| Chloroazodine | 250 mg |
| Lactose | 1 g |

Cette poudre peut être conservée en sachet et dissoute dans 1 l d'eau.

### Exemple 2

### Comprimés vaginaux

On mélange, sous forme pulvérulente, de la chloroazodine, de l'acide tartrique et du bicarbonate de sodium, dans des proportions appropriées en pharmacie, puis on comprime le tout sous la forme de comprimés vaginaux.

Des essais expérimentaux ont été effectués par le laboratoire de l'Institut Pasteur du Brabant pour examiner l'efficacité d'un des agents suivant l'invention.

Ces essais comprennent comme matériel de départ :
- de la N, N'-dichloroazodicarbonamidine (chloroazodine) à 95 % pure. La pureté a été examinée par chromatographie en couche mince et par un test de carbone-hydrogène (v. BRAZ G.I. et cons., J. Applied Chem. (USSR) 17,565-9 (1944)).
- un surnageant de cultures de cellules (Molt-3) continuellement productrices de virus VIH-1. Le surnageant viral utilisé a une activité de transcriptase inverse de 1 x 10⁶ cpm/ml.
- des cellules d'une lignée T humaine continue de phénotype T₄ (Supt-1) cultivée en milieu RPMI, additionné de 10 % de sérum de veau foetal et de 1 % de glutamine.

L' efficacité de la chloroazodine va être étudiée à deux niveaux :
**a)** action sur l'infectivité du virus VIH-1 pour la lignée Supt-1.
**b)** action sur l'intégration du génome viral dans l'ADN chromosomial des cellules Supt-1.

Les résultats de ces essais sont donnés ci-dessous dans les Exemples 6 à 8.

### Exemple 3

Essai de toxicité de la chloroazodine vis-à-vis des cellules Supt-1.

La chloroazodine à la dilution de 1/500, ce qui correspond à 2 mg/ml, est toxique pour les cellules Supt-1. Les dilutions de 1/1500 et de 1/3000, ce qui correspond à des concentrations de 660 µg/ml et de 330 µg/ml, ne diminuent par contre pas la viabilité cellulaire.

### Exemple 4

Infectivité du virus VIH-1 pour la lignée Supt-1.

On fait incuber des préparations virales de VIH-1 à haut titre en présence de dilutions de chloroazodine de 1/1500 et de 1/3000 et pendant différents temps d'incubation. Pendant 30 minutes à 37°C, on traite préalablement les cellules Supt-1 par 10 µg/ml de polybrène, puis on les inocule avec les préparations de virus traités. On prévoit comme témoins, des cellules non inoculées, et des cellules inoculées par une préparation de virus non traités.

On détermine ensuite l'infectivité, à chaque passage cellulaire, de ces préparations en suivant la production virale (mesure de l'antigène p24 exprimé) dans les lysats cellulaires solubilisés des cellules Supt-1 examinées.

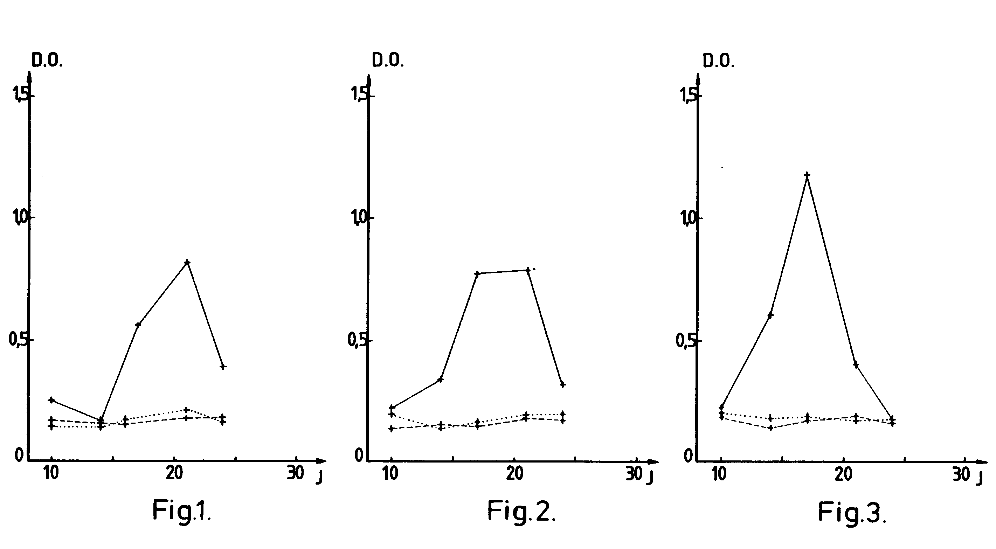

Les mesures du pouvoir infectieux du virus VIH-1 après incubation avec de la chloroazodine ressortent des tableaux 1 à 3 suivants, ainsi que des figures 1 à 3 qui correspondent aux tableaux 1 à 3. Sur ces figures, on trouve en ordonnée la densité optique (D.O.) et en abscisse le nombre de jours après l'infection. Le trait plein représente le témoin non traite, le trait interrompu la dilution de 1/1500 de chloroazodine, et le trait pointillé la dilution de 1/3000 de chloroazodine.

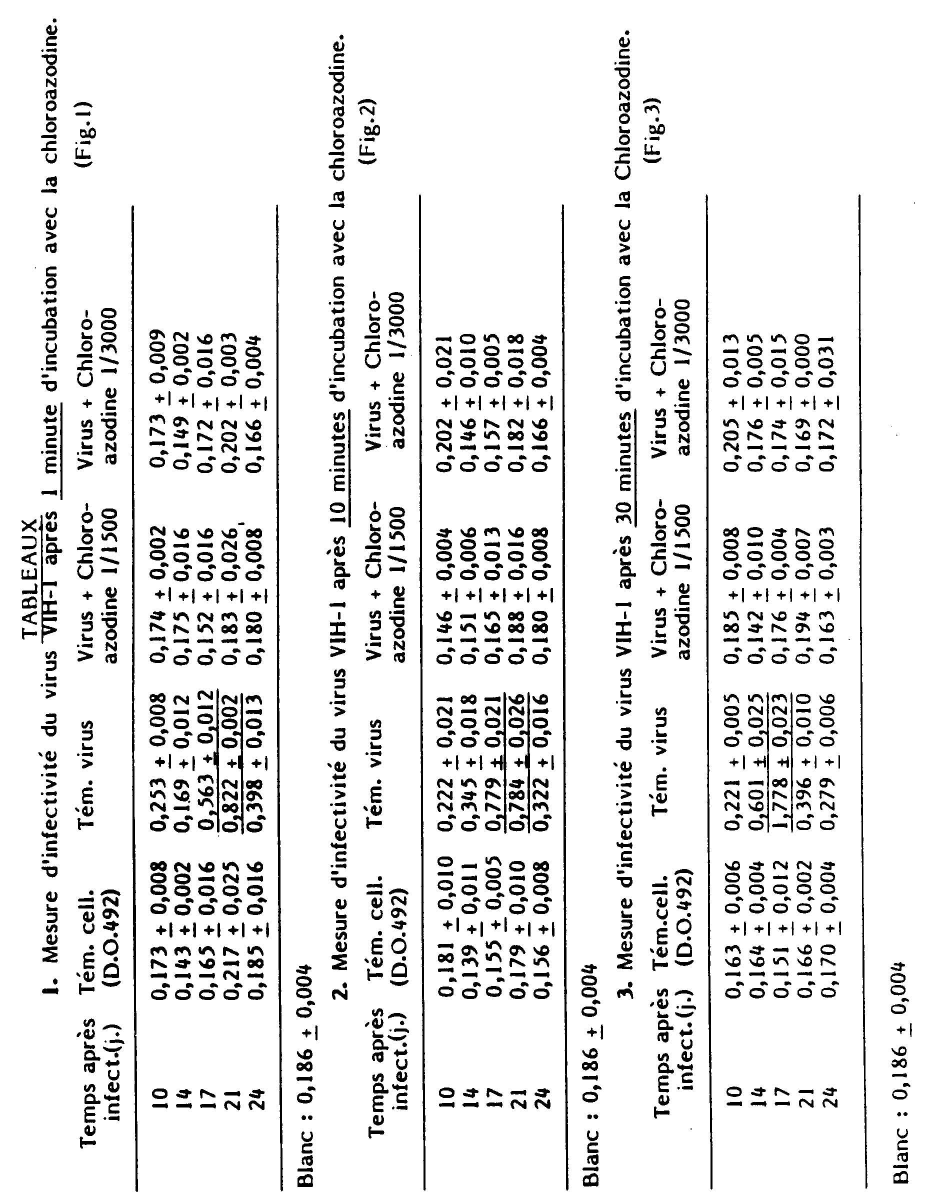

Il ressort de cet essai que la chloroazodine aux dilutions de 1/1500 et 1/3000 empêche totalement le virus VIH-1 de se multiplier dans les cellules Supt-1 et ceci après 1 minute, 10 minutes et 30 minutes d'incubation avec l'agent actif, à la température ordinaire.

Au microscope en contraste de phase, on n'a par ailleurs observe aucun effet cytopathogène pour les cellules inoculées avec du virus prétraité à la chloroazodine. Par contre des syncytia apparaissent dès le 14ème jour après l' infection avec la préparation virale témoin.

### Exemple 5

Intégration du génome viral dans l'ADN chromosomial des cellules Supt-1.

Par amplification génétique grâce à une polymérase thermostable, on met en évidence la présence des gènes "gag", "LTR" et "env" du provirus VIH-1. Cette technique est appelée P.C.R. (Polymerase Chain Reaction) (CHIN-YIH OU et cons., Sciences, 239, 295-297, 1988).

Cette technique est appliquée pour la détection du provirus VIH-1 dans le génome des cellules Supt-1 inoculées par du virus VIH-1 incubé avec de la chloroazodine, comme décrit dans l'exemple 4.

Des oligonucléotides amplifiés et visibles aux UV grâce à du bromure d'éthidium apparaissent uniquement dans les tubes à essais qui correspondent à une inoculation par du virus VIH-1 non traité à la chloroazodine.

De même, après hybridation avec des sondes spécifiques pour les trois gènes recherchés, sondes marquées au P³², on peut conclure à l'absence totale de provirus VIH-1 dans les cellules Supt-1 ayant reçu comme inoculats des virions VIH-1 traités à la chloroazodine aux dilutions de 1/1500 et 1/3000.

Il ressort par conséquent clairement des essais des exemples 3 à 5 que l'éradication des virus par la chloroazodine à faible concentration est totale dans les cellules traitées en un temps très bref (1 minute ou moins). Il est important de noter que cette activité remarquable de la chloroazodine a lieu en milieu organique.

### Exemple 6

Des lymphocytes humains provenant du sang périphérique de donneurs séro-négatifs (PBL) sont infectés par du VIH-1 provenant du surnageant de cellules Molt-3, c'est-à-dire de cellules continuellement productrices de ces virus.

Au jour 8 de l'infection, celle-ci est contrôlée par l'expression de l'antigène p 24 (Test Elisa, voir Exemple 4).

Quatre populations cellulaires sont étudiées quant à leur mortalité :
1 des lymphocytes normaux en culture (Témoin A).
2 des lymphocytes normaux en présence de chloroazodine 1/3000.
3 des lymphocytes infectés non traités (Témoin B).
4 des lymphocytes infectés, en présence de chloroazodine 1/3000.

**Tableau 4**

| Cellules | Durée (min.) | Nombre de cellules vivantes | Nombre de cellules mortes | % de cellules mortes |
|---|---|---|---|---|
| Témoins A | 1 | 21 | 20 | 49 % |
| | 10 | 26 | 20 | 43 % |
| | 30 | 22 | 16 | 42 % |
| | 180 | 16 | 22 | 58 % |
| Lymphocytes + chloroazodine 1/3000 | 1 | 15 | 20 | 57 % |
| | 10 | 24 | 20 | 45 % |
| | 30 | 18 | 18 | 50 % |
| | 180 | 18 | 16 | 47 % |
| Témoins B | 1 | 19 | 15 | 44 % |
| | 10 | 20 | 17 | 46 % |
| | 30 | 16 | 23 | 59 % |
| | 180 | 12 | 19 | 61 % |
| Lymphocytes infectés + chloroazodine 1/3000 | 1 | 17 | 31 | 65 % |
| | 10 | 14 | 34 | 71 % |
| | 30 | 21 | 39 | 54 % |
| | 180 | 8 | 20 | 71 % |

Les comparaisons montrent que les lymphocytes infectés sont, en présence de chloroazodine, détruits plus fortement. En effet les différences entre les témoins A et B et les lymphocytes sains + chloroazodine sont non significatives, alors que les cultures de lymphocytes infectés voient leur mortalité accrue de 15 % en présence de chloroazodine.

On peut donc conclure à l'existence d'une toxicité sélective de la chloroazodine pour des cellules infectées.

### Exemple 7

Des cellules Molt-3 B, continuellement productrices de virus VIH, ont été traitées par de la chloroazodine à des concentrations de 1/1500 et 1/3000 pendant des périodes de 1 minute, 10 minutes et 30 minutes.

Les antigènes p 24 exprimes dans les surnageants de culture ont été évalués après 24 h., 48 h., 72 h. et 96 h.

Pour mesurer cet effet en contournant la toxicité du produit pour les cellules infectées, on a choisi de prendre un nombre cellulaire extraordinairement élevé de manière à conserver un nombre très faible, mais suffisant, de cellules vivantes après 96 h.

**Tableau 5**

| Mesure du pouvoir infectieux des virus VIH-1 dans des cellules Molt-3 B (densité optique). | | | | | | | | |
|---|---|---|---|---|---|---|---|---|
| Temps après traitement | Témoin cellulaire sans virus | Cellules Molt-3 B | Cellules Molt-3 B + chloroazodine 1/1500 | | | Cellules Molt-3B + chloroazodine 1/3000 | | |
| | | | Durée du traitement (min) | | | Durée du traitement(min) | | |
| | | | 1 | 10 | 30 | 1 | 10 | 30 |
| 24 h. | 0,174 | 0,938 | 0,175 | 0,182 | 0,194 | 0,247 | 0,180 | 0,211 |
| 48 h. | | 0,802 | 0,175 | 0,181 | 0,155 | 0,154 | 0,176 | 0,162 |
| 72 h. | | 0,743 | 0,167 | 0,145 | 0,173 | 0,170 | 0,152 | 0,176 |
| 96 h. | | 1,180 | | | | | | |

En conclusion, la chloroazodine est capable d'empêcher la production de VIH par des cellules infectées et en cas de repopulation par des cellules non infectées de permettre, après un laps de temps de 96 heures, d'obtenir des cultures non infectées.

### Exemple 8

On a reproduit l'expérience de l'exemple 7, mais avec des lymphocytes humains infectés par du VIH-1. La chloroazodine a été utilisée à la dose de 1/3000 pendant 1 heure, 3 heures et 6 heures sur les cellules infectées.

**Tableau 6**

| Mesure du pouvoir infectieux des virus VIH-1 dans des lymphocytes humains infectés (densité optique). | | | | | |
|---|---|---|---|---|---|
| Temps après traitement | Témoin cellulaire sans virus | Témoin cellulaire + virus | Lymphocytes + chloroazodine 1/3000 | | |
| | | | Durée du traitement (heures) | | |
| | | | 1 | 3 | 6 |
| 4 jours | 0,184 | 0,421 | 0,170 | 0,197 | 0,192 |
| 7 jours | 0,199 | 0,390 | 0,170 | 0,158 | 0,203 |
| 11 jours | 0,072 | 0,461 | 0,114 | 0,095 | 0,078 |

Les même conclusions que celles déduites de l'exemple 7 peuvent être reproduites.

Afin de conserver un nombre suffisant de lymphocytes vivants il a fallu limiter l'expérimentation à 6 heures.

### Exemple 9

On a évalué la toxicité de la chloroazodine à 330 µg/ml et de la chloroazodine associée à un tensio-actif quaternaire sur l'environnement, par évaluation de la toxicité aiguë sur le poisson.

Le produit, tel que prévu, lorsqu'il est utilisé pour la désinfection, ne peut atteindre le seuil de toxicité pour l'environnement mesuré et il peut donc être considéré comme sûr.

### Exemple 10

Composition désinfectante.

On a préparé une composition désinfectante contenant, en plus de la chloroazodine, un mélange désinfectant de bromures d'alkyltriméthylammonium, dénommé cetrimide. Une étude de synergie a montré que cette composition était capable d'inhiber complètement en 5 minutes à 25°C, en présence de 10 % de sérum de veau foetal, les germes suivants : Bacillus cereus, Candida albicans, Escherichia coli, Klebsiella pneumoniae, Streptococcus pyogenes, Mycobacterium fortuitum, Proteus vulgaris, Pseudomonas aeruginosa, Salmonella groupe B, Enterobacter agglomerans, Staphylococcus aureus, les virus Herpes simplex I, les virus de la stomatite vésiculaire, les rétrovirus, Aspergillus fumigatus, Microsporum canis, Trichophytor rubrum.

Sur base de cette constatation surprenante, une composition de comprimé à activité effervescente, même dans le sang et les matières organiques, a été mise au point.

| | |
|---|---|
| Chloroazodine | 330 mg |
| Bromure d'alkyltriméthylammonium (cetrimide) | 100 mg |
| Acide citrique | 150 mg |
| Acide tartrique | 175 mg |
| NaHCO₃ | 375 mg |
| Avicel PH102 | 180 mg |
| Lactose EFK | 522 mg |
| Aerosil 200 | 3,8 mg |
| Benzoate de dénatonium | 0,2 mg |
| pour 1 comprimé | 1836 mg |
| Dose : 1 comprimé pour 1 litre d'eau. | |

Il doit être entendu que la présente invention n'est en aucune façon limitée aux formes de réalisation décrites ci-dessus et que bien des modifications peuvent y être apportées sans sortir de son cadre.

De nombreuses autres compositions désinfectantes ou pharmaceutiques peuvent être prévues en dehors de celles données à titre d'exemple, en suivant uniquement les formulations généralement appliquées dans le domaine concerné, qu'il s'agisse de produits de nettoyage, de compositions cosmétiques, de médicaments ou autres.

## Revendications

1. Utilisation d'une composition de désinfection contenant, comme agent actif, un dérivé N-chloro d'amines condensées provenant de dérivés de guanidine en une concentration à laquelle il ne présente pas de toxicité lorsque la composition vient en contact avec une peau ou des muqueuses d'un être humain ou d'un animal, ainsi qu'un support approprié, et éventuellement d'autres agents désinfectants ou adjuvants courants, pour la désinfection, vis-à-vis des virus du groupe rétrovirus, en particulier des virus d'immunodéficience humaine VIH, d'objets inanimés.

2. Utilisation d'une composition de désinfection contenant, comme agent actif, de la chloroazodine (N,N'-dichloroazodicarbonamidine) en une concentration à laquelle elle ne présente pas de toxicité lorsque la composition vient en contact avec une peau ou des muqueuses d'un être humain ou d'un animal, ainsi qu'un support approprié, et éventuellement d'autres agents désinfectants ou adjuvants courants, pour la désinfection, vis-à-vis des virus du groupe rétrovirus, en particulier des virus d'immunodéficience humaine VIH, d'objets inanimés.

3. Utilisation suivant la revendication 2, caractérisée en ce que la composition comprend une solution aqueuse de chloroazodine présentant une concentration en ce composé de 300 µg/ml à 1 mg/ml, en particulier de 330 µg/ml à 660 µg/ml.

4. Utilisation suivant la revendication 2, caractérisée en ce que la composition comprend de la chloroazodine et, comme autre agent désinfectant ou adjuvant, du cétrimide ou un laurylsulfate.

5. Utilisation suivant l'une des revendications 1 à 5, dans laquelle les objets inanimés à désinfecter sont des matériels sanitaires en matière plastique, textile ou caoutchouteuse, des instruments, appareils et vêtements médicaux et vétérinaires, des objets nécessitant une manipulation dans le domaine de l'alimentation, des instruments de cosmétologie, des véhicules sanitaires, des surfaces de sol et de paroi, des appareils sanitaires et hygiénique, des eaux de piscine, des boissons, et des objets analogues.

6. Utilisation suivant l'une des revendications 1 à 5, dans laquelle les objets inanimés à désinfecter sont des excréments, des résidus d'analyses de laboratoire, des échantillons prélevés d'un corps humain ou animal, et des objets analogues.

7. Utilisation suivant l'une des revendications 1 à 5, dans laquelle les objets inanimés à désinfecter sont des compositions cosmétiques.

8. Utilisation suivant l'une des revendications 1 à 5, dans laquelle les objets inanimés à désinfecter sont des produits susceptibles d'entrer en contact avec la peau et les muqueuses d'un corps humain ou animal, éventuellement porteur de virus, produits dans lesquels ou sur lesquels ledit agent ou composition de désinfection forme simultanément un milieu de barrière à la transmission des virus précités.

9. Utilisation suivant la revendication 8, dans laquelle les produits susceptibles d'entrer en contact avec la peau ou les muqueuses, sont des gants de chirurgien ou de dentiste, des pessaires, des condoms, et des objets analogues.

10. Utilisation d'un dérivé N-chloro d'amines condensés provenant de dérivés de guanidine, pour la fabrication d'une composition thérapeutique à mettre en oeuvre dans le traitement ou la prophylaxie des infections par les virus du groupe rétrovirus, et en particulier pour les infections par les virus d'immunodéficience humaine VIH.

11. Utilisation de chloroazodine, pour la fabrication d'une composition thérapeutique à mettre en oeuvre dans le traitement ou la prophylaxie des infections par les virus du groupe rétrovirus, et en particulier pour les infections par les virus d'immunodéficience humaine VIH.

## Claims

1. Use of a disinfecting composition comprising, as active agent, a N-chloro derivative of condensed amines from guanidine derivatives with a concentration at which said agent has no toxicity when the composition comes in contact with a skin or mucosas of a human or animal body, as well as an appropriate carrier, and optionally other disinfecting agents or usual additives, for disinfecting inanimate objects with respect to viruses of retrovirus group, particularly the human immunodeficiency viruses HIV.

2. Use of a disinfecting composition comprising, as active agent, chloroazodin (N,N'-dichloroazodicarbonamidine) with a concentration at which said agent has no toxicity when the composition comes in contact with a skin or mucosas of a human or animal body, as well as an appropriate carrier, and optionally other disinfecting agents or usual additives, for disinfecting inanimate objects with respect to viruses of retrovirus group, particularly the human immunodeficiency viruses HIV.

3. Use according to claim 2, characterized in that the composition contains an aqueous solution of chloroazodin having a chloroazodin concentration of 300 µg/ml to 1 mg/ml, particularly of 330 µg/ml to 660 µg/ml.

4. Use according to claim 2, characterized in that the composition comprises chloroazodin and, as other disinfecting agent or additive, cetrimide or a laurylsulfate.

5. Use according to any one of claims 1 to 4 wherein the inanimate objects to disinfect are plastic, rubber or textile sanitary materials, medical or veterinary instruments, devices and clothes, objects requiring a handling in the alimentary field, cosmetology instruments, sanitary vehicles, ground and wall surfaces, sanitary and hygienic apparatuses, water for swimming pool, beverages, and the like.

6. Use according to any one of claims 1 to 4, wherein the inanimate objects to disinfect are excrements, waste of analysis laboratories, samples taken off from a human or animal body, and the like.

7. Use according to any one of claims 1 to 4, wherein the inanimate objects to disinfect are cosmetic compositions.

8. Use according to any one of claims 1 to 4, wherein the inanimate objects to disinfect are products which can come into contact with the skin and the mucosas of a human or animal body, optionally a virus carrier, products in which or on which said disinfecting agent or composition simultaneously forms a barrier medium against the transmission of said viruses.

9. Use according to claim 8, wherein said products which can come into contact with the skin and the mucosas are surgeon or dentist gloves, pessaries, preservative sheaths and similar articles.

10. Use of a N-chloro derivative of condensed amines from guanidine derivatives for manufacturing a therapeutic composition to be used in the treatment or the prophylaxis of infections by the viruses of retrovirus group, particularly the infections by the human immunodeficiency viruses HIV.

11. Use of chloroazodin, for manufacturing a therapeutic composition to be used in the treatment or the prophylaxis of infections by the viruses of retrovirus group, particularly the infections by the human immunodeficiency viruses HIV.

## Patentansprüche

1. Verwendung eines Desinfektionsmittels, enthaltend als Wilkstoff ein kondensiertes N-Chloramin-Derivat, das von Guanidin-Derivaten stammt; in einer Konzentration, in der es keine Toxizität aufweist, wenn das Mittel mit der Haut oder den Schleimhäuten eines Maschen oder eines Tiers in Kontakt kommt, sowie ein geeigneter Träger und gegebenfalls andere Desinfektionsmittel oder übliche Hilfsstoffe, zur Desinfektion von leblosen Objekten gegenüber Viren aus der Gruppe der Retroviren, insbesondere von humanen Immundefizienz-Viren (HIV).

2. Verwendung eines Desinfektionsmittels, enthaltend als Wirkstoff Chlorazodin (N,N'-Dichlorazodicarbonamidin) in einer Konzentration, in der es keine Toxizität aufweist, wenn das Mittel mit der Haut oder den Schleimhäuten eines Menschen oder eines Tiers in Kontakt kommt, sowie ein geeigneter Träger und gegebenenfalls andere Desinfektionsmittel oder übliche Hilsstoffe, zur Desinfektion von leblosen Objekten gegenüber Viren aus der Gruppe der Retroviren, insbesondere von humanen Immundefizienz-Viren (HIV).

3. Verwendung nach Anspruch 2, dadurch gekennzeichnet, daß die Zusammensetzung eine wäßrige Lösung des Chlorazodins in einer Konzentration an dieser Verbindung von 300 µg/ml bis 1 mg/ml, insbesondere von 330 µg/ml bis 660 µg/ml umfaßt.

4. Verwendung nach Anspruch 2, dadurch gekennzeichnet, daß die Zusammensetzung Chlorazodin und als weiteres Desinfektionsmittel oder als Hilfsstoff Cetrimid oder ein Laurylsulfat umfaßt.

5. Verwendung nach einem der Ansprüche 1 bis 4, wobei die zu desinfizierenden, leblosen Objekte Sanitärmaterialien aus Kunststoff, Texil oder Kautschuk, medizinische sowie tiermedizinische Instrumente, Apparate und Kleidung, Objekte aus dem Lebensmittelbereich die eine Bearbeitung erfordern, kosmetische Instrumente, Sanitätsfahrzeuge, Boden- und Wandoberflächen, Sanitär- und Hygienegeräte, Badewasser, Getränke und entsprechende Objekte darstellen.

6. Verwendung nach einem der Ansprüche 1 bis 5, wobei die zu desinfizierenden, leblosen Objekte Exkremente, Analysenrückstände aus Laboratorien, aus Mensch- oder Tierkörpern entnommene Proben und entsprechende Objekte darstellen.

7. Verwendung nach einem der Ansprüche 1 bis 5, wobei die zu desinfizierenden, leblosen Objekte kosmetische Zusammensetzungen darstellen.

8. Verwendung nach einem der Ansprüche 1 bis 5, wobei die zu desinfizierenden, leblosen Objekte Produkte darstellen, die mit der Haut oder den Schleimhäuten des menschlichen oder tierischen Körpers in Kontakt kommen können, die gegebenenfalls Träger von Viren sein können, wobei in den oder auf den Produkten dieses Desinfektionsmittel, oder die Zusammensetzung zur Desinfektion gleichzeitig einen Barrierenbereich für die Übertragung der vorgenannten Viren erzeugt.

9. Verwendung nach Anspruch 8, wobei die Produkte, die mit der Haut oder den Schleimhäuten in Kontakt kommen können, Handschuhe von Chirurgen oder Zahnärzten. Pessare, Kondome und entsprechende Objekte darstellen.

10. Verwendung eines kondensierten N-Chloramin-Derivats, das von Derivaten des Guanidins stammt, zur Herstellung eines Heilmittels zur Behandlung oder zur Vorbeugung von Infektionen durch Viren aus der Gruppe der Retroviren, und insbesondere von Infektionen durch den humanen Immundefizienz-Virus (HIV).

11. Verwendung von Chlorazodin zur Herstellung eines Heilmittels zur Behandlung oder zur Vorbeugung von Infektionen durch Viren aus der Gruppe der Retroviren, und insbesondere von Infektionen durch den humanen Immundefizienz-Virus (HIV).
